# EUROPEAN PATENT APPLICATION

(11) **EP 2 394 700 A1**
(43) Date of publication of application: **14.12.2011**
(21) Application number: 10186743.0
(22) Date of filing: 06.10.2010
(51) Int. Cl.: A61N 5/06

(54) **Device for irradiating tissue**

(30) Priority: 08.06.2010 EP 10165299
(71) Applicant: A.R.C. Laser GmbH, 90411 Nürnberg (DE)
(72) Inventor: Thyzel, Reinhold, 90542 Eckenhaid (DE)
(74) Representative: Schröer, Gernot H.

(57) **Abstract**

The present application is directed to a device 1 for irradiating human or animal tissue, in particular nerves, with laser light. In order to obtain gentle and effective destruction of tissue, the device 1 comprises a laser light source L adapted to generate laser light in a wavelength between 690 nm and 710 nm, and a light guide 3 for guiding through the laser light. A first end 3a of the light guide 3 is coupled the laser light source L and a second end 3b of the light guide 3 is coupled to an applicator 2 adapted to apply the laser light to the tissue.

## Description

The present application is directed to a device for irradiating human or animal tissue, in particular nerves, with laser light.

Such devices are known for example as deep tissue medical lasers for irradiating tissue, such as nociceptive (pain) nerves, lying up to several centimetres below the outer skin of the human or animal body. Such lasers are for example used to destroy nerve cells that continuously generate acute pain conditions with a human or animal being.

Further, low level laser therapy (LLLT) devices are known which obtain pain release for nociceptive nerves, for example, by processes such as vaso-dilatation or by inducing metabolic changes.

It is an object of the invention to provide a device for irradiating human or animal tissue, that is effective in gently destroying tissue, and preferably can be used for deep tissue applications.

This object is achieved by providing a device which is adapted and meant (or: provided) for irradiating human or animal tissue, in particular nerves, nerve tissue, and nerve cells, particularly nociceptive nerve cells, in human or animal tissue with laser light which device comprises at least one laser light source which is adapted and designed or can be set to generate laser light having a wavelength between 690 nm and 710 nm, in particular between 701 nm and 705 nm, preferably 703 nm.

Further, the device comprises a light guide for guiding through laser light generated by at least one of the at least one laser light source. A first end of the light guide is coupled or connected to or adapted to being coupled or connected to a laser light exit or exit site of the at least one laser light source. Hence, laser light can be guided from the exit site via the light guide to remote locations.

The device further comprises at least one applicator, preferably of handheld-type, adapted or designed to apply the laser light to the tissue, to a laser light application site for example. The applicator at least one of contains, holds, is coupled, is connected and is connectable to at least a second end of the light guide. Via the applicator, which may comprise a needle, the laser light can be applied even to deep tissue, in particular nerves or nerve cells, lying up to several centimetres or even further blow the outer skin of the human or animal being for example.

The laser light with the present device in the specific wavelength spectrum between 690 nm and 710 nm, in particular between 701 nm and 705 nm, preferably 703 nm, surprisingly provides or causes a very efficient and nevertheless gentle selective destruction or disruption or at least deactivation of tissue, in particular nerve cells, particularly nociceptive nerve cells, without significant damage to the surrounding tissue or the tissue structure as such. In contrast to known deep tissue treatment devices, which are generally based on the concept cell disruption by heat generation and which disrupt tissue without any specificity or low level laser therapy devices, which may ease pain by vaso-dilatation or metabolic changes, tissue disruption or deactivation with the present device according to the invention is based on selective absorption and cell resonance phenomena. These phenomena make it possible to selectively destroy and disrupt respective cells, in particular to cause death of respective cells while leaving surrounding tissue basically intact.

Specific death or disruption of tissue, particularly pain nerves, as obtained with the proposed device according to the invention, has the advantage of providing long term pain relief, for example for many months. Note that this is not the case with known low level laser therapy methods which generally can not assure long term pain relief.

The obtained selective disruption is inter alia achieved by using laser light wavelength lying in the range of 690 nm to 710 nm. Nerve tissue, in particular nociceptive nerve cells or nerves, in particular C-fibres and/or myelin, can be disrupted best if a wavelength of about 703 nm (+/- 2 nm) is applied directly and selectively to the respective tissue, the area of pain source for example.

Due to selective destruction or disruption, surrounding tissue is virtually left unaffected and not thermally disintegrated or destroyed. Disruption with the proposed device is, at least for nerve tissue, based on absorption and cell resonance. Commonly, nerve tissue consists of lipids as predominant component which greatly absorb the above-identified wavelengths whereas surrounding non nervous tissue generally does not.

Using lasers having low average output power and operable at high pulsation rates, the amount of heat generated, if at all, is not detrimental to surrounding tissue. Hence, surrounding tissue can be kept from being affected or damaged. The phenomena of absorption and cell resonance can be achieved with laser light intensities as low as 0.5 mW to 10 mW, in particular between 1 mW and 6 mW, at least for nociceptive tissue, particularly nerves or nerve cells.

It shall be mentioned that all medical lasers for tissue laser treatment known so far do not make use of laser light having wavelength as set out above.

The proposed device can therefore be used for deep tissue low intensity laser treatment or therapy. In particular, the device can be used for deep tissue low intensity laser ablation and deep tissue low intensity laser neuroablation.

In a preferred embodiment, the light guide comprises an optical fiber. Optical fibers are effective in guiding through laser light, in particular laser light of the aforementioned wavelengths. The optical fiber preferably has a diameter between 0.15 mm and 0.7 mm. Such a diameter range is generally sufficient to disrupt pain fibers which are responsible for permanent or chronic acute pain conditions. Depending on the spot size of tissue to be treated, diameter ranges from 0.15 mm and 0.5 mm, or 0.2 mm to 0.3 mm may be used.

A further advantage of using such diameters is that such optical fibers can be inserted or embedded into conventional needles or cannulaes generally available in most medical practices or hospitals. By using such needles or cannulaes it is possible to bring the second end of the light guide, in particular of the optical fiber, close to the tissue to be irradiated leading to enhanced selective tissue destruction. Further, the use of such needles or cannulaes makes it possible to provide minimally invasive deep tissue low intensity laser treatment or therapy.

If clinical diagnosis is important to determine the area of treatment, the treatment preferably is conducted by a physician or equivalent professional community. However, as low level laser therapy (LLLT) already has a widespread use, various models and designs will be possible for both the general public and the professional community alike.

As already indicated above, the applicator can comprise at least one hollow needle or cannula. Such a needle or cannula , preferably comprises a tip cut, i. e. a sharp front tip or edge, adapted to open or cut tissue in the course of inserting the needle or cannula into a human or animal body for example, which tissue lying in front of or ahead of the tissue to be irradiated.

Especially in connection with minimally invasive treatments, the hollow needle preferably has an inner diameter of about or less than 0.6 mm, preferably of about or less than 0.5 mm. Hence, conventional fine needles of 22G (22 Gauge) or 25G (25 Gauge) or other size can be used. In such cases, the light guide, especially the optical fiber, preferably has a diameter of 0.2 mm or 0.3 mm such that it can be inserted or is insertable into the needle cannula. Note that the device may be adapted to using light guides of different diameters without requiring any constructive or structural amendments, of the applicator for example.

Using the aforementioned types of fine needles makes it possible to reach tissue areas that lie in deeper layers of the human or animal body, i. e. remote from the human or animal body surface. In particular a spinal needle may be used through which or by which the light guide can be inserted. The concept of using conventional needles has the advantage that immense cost savings compared to heavier and bigger non-needle like conduits as used with known deep tissue medical laser treatment devices can be obtained.

In a preferred embodiment, the light guide is embedded in the needle cannula, preferably fixed therein in a non-detachable manner, for example by gluing. In this case the needle and light guide preferably make up one non detachable unit. Fixing the light guide to the needle, for example by gluing or other suitable means, has the advantage that the laser light can be applied to the tissue more precisely and predictable, as in particular relative movement between the light guide and the needle in the course of inserting the needle into the human or animal body can be avoided. In particular such arrangements allow to specifically irradiate predetermined areas of the spine.

In order to establish easy and quick connection between the light guide and at least one of the at least one laser light source and applicator, either one or both of them may comprise a locking or latching mechanism, in particular a conventional Luer-locking mechanism. Luer-locking mechanisms are quite common and allow easy attachment or connection of different sizes of syringes to various needle sizes. Providing a Luer-locking mechanism to the at least one laser light source makes it also possible to attach common spinal needles or other needles, facilitating the use of existing needle systems. Locking or latching mechanisms may also be beneficial for fixing the light guide to the needle thereby preventing relative movement during needle insertion and laser light application.

For low intensity laser treatment it is of particular advantage if at least one of the at least one laser light source is adapted to emit laser light with an output average power between 0.5 mW to 10 mW, in particular between 1 mW and 6 mW, preferably between 4 mW and 6 mW such as 5 mW (+/- 0.8 mW). It shall be mentioned that laser light sources having output average powers of less than 5 mW fall under laser classification 3R or below.

The laser light may be a pulsed laser light with a pulse length in the order of nanoseconds to picoseconds or less, in particular 0.1 to 50 ns, preferably 25 ns. Further, at least one of the at least one laser light source may be operated at an output peak power of 30 nW to 50 nW (+/-8nW).

In using proposed low intensity laser light sources mentioned beforehand a non-selective destruction of the tissue exposed to the laser light can be avoided. The latter problems actually occur with conventional deep tissue lasers which usually have average output powers of about 100 mW and above.

In a further embodiment, the device may comprise a timer unit for respectively activating and inactivating respective laser light source, in timing intervals in the range between 1 s to 30 s, in particular 3 s to 7 s. The timing intervals may be selected according to respective low intensity laser treatment requirements with regard to the tissue of concern.

In order to allow easy operation, the device may comprise an exchangeable and/or rechargeable source of energy, in particular a rechargeable battery source of energy, for powering at least one of the at least one the laser light source. Here, the device can be operated without restrictions imposed by cable connections and the like otherwise needed for powering the device. Such a source of energy may be used with the proposed device, as laser light is applied at comparatively low intensities.

In a yet further embodiment, the device comprises a casing accommodating therein at least one of the source of energy and the at least one laser light source. The casing may be of handheld-type allowing a user to freely operate the whole device without any restrictions as to cable connections, in particular electric power supply and/or data exchange lines. The casing may accommodate exact one laser light source, or, as the case may be, several laser light sources. In the case that several laser light sources are provided they may be of different type, in particular with respect to output power and/or other operational properties.

The device, in particular the casing, may be adapted to enable easy exchange of the source of energy and/or laser light source/s. Such an exchange may for example be required in case of failure of respective components. It is also conceivable that at least one of the at least one laser light source may be exchanged if respective treatment or therapy so requires.

The type of laser light source accommodated within the casing may be visually indicated on a display, or via other visualization means, such as light emitting diodes possibly in connection with respective labelling provided on the casing for example.

In particular in the case of several laser light sources, and, as the case may, be several means for coupling laser light into the light guide, which laser light sources are jointly accommodated in the casing, the device may be adapted such that a respective laser light source is selected according to respective irradiation requirements of a given or selected treatment program. A respective laser light source may be automatically selected by the device and/or may be selected by a user, via a user interface for example. Further, it is possible that a suitable laser light source is selected according to respective treatment mode or treatment program selected by a user and/or the device. For security reasons, automated selection of a suitable laser light source by the device may require user confirmation.

The casing may accommodate optical coupling means for effectively coupling laser light emitted by at least one of the at least one laser light source into the light guide. There may be provided several means for coupling laser light into the light guide and differing in coupling characteristics. Respective coupling characteristics may be selected to account for different types of laser light sources and/or different types of light guides.

Further, the casing may comprise a user interface adapted to allow a user to operate the device in at least one, preferably in all possible, modes of operation. In the simplest case, the user interface may comprise a button for activating and deactivating the laser light source/s. In a more elaborate embodiment, the user interface may comprise a control panel, comprising several buttons and/or display elements and/or touch sensitive elements, in particular touch sensitive display elements, for example.

The control panel may be adapted to allow adjustment of at least one, preferably of any, operational parameter of the device. Such a user interface makes it possible to operate the device without the need for establishing cable connections to remote controllers or the like. Further, the progress and/or actual operational parameters may be displayed on a display site of the user interface.

The casing may also accommodate an electronic board, comprising a controller, a microcontroller for example or the like, adapted to operate the device, in particular according operational parameters and/or operational modes selected by a user and/or automatically set by the device.

Further, the casing may accommodate a memory which may be adapted to store operational parameters and/or operational modes selected and/or to be selected by a user and/or to provide a selection of preset operational parameters, operational modes and/or operational programs. In this case the controller may access data stored in the memory for operating the device and/or for setting up an operational program and/or to process and prepare a parameter selection presented to a user via display elements of the user interface.

In an embodiment, the casing may be a non-conducting insulated casing. In this case a separate grounding at least during charging and/or operation of the device is not mandatory. The non-conducting casing avoids external disturbances due to electrostatic charges of an operator, for example. Further, an operator can be protected against any voltage or electric current induced impacts, e. g. electric shocks, during operating the device and/or in the course of charging the battery. The casing can be designed to accommodate therein further components such as the laser light source/s, electronic units, units required for charging the battery and the like.

The casing may comprise coupling means for coupling or connecting thereto an applicator. The coupling means may comprise a standardized connector used in the field, as for example a Luer-lock or other suitable locking or connecting mechanisms, in particular quick connectors.

The coupling means provided with the casing and/or optical elements accommodated within the casing and adapted to couple light from the at least one laser light source into the light guide may be adapted such that optimal coupling between respective laser light source and light guide is automatically established upon connecting the applicator to the housing. Preferably, the coupling means and optical elements are adapted such that no further manual adjustment, in particular of optical coupling parameters, is required after establishing connection between the applicator and the casing.

In particular with respect to selective local disruption of tissue, it is of advantage if the applicator or second end of the light guide is adapted to apply the laser light to an area of about the cross-section of the light guide, preferably with a scatter radius of 1 mm at the most. Preferably, the laser spot applied to the tissue of concern is confined to the light guide diameter with less than 1mm scatter.

Embodiments of the invention are now described in connection with the annexed figures, in which
- Fig. 1: shows an embodiment of a device for irradiating tissue;
- Fig. 2: shows a detail of the device according to Fig. 1;
- Fig. 3: shows a further detail of the device according to Fig. 1;
- Fig. 4: shows further detail of the device according to Fig. 1;
- Fig. 5: shows a detail of Fig. 4;
- Fig. 6: shows a detail of Fig. 5;
- Fig. 7: shows in a transparent-type representation a further embodiment of a device for irradiating tissue; and
- Fig. 8: shows in a cross-sectional view an applicator of Fig. 7.

Throughout the figures, like elements are denoted by like reference signs. The figures may not be true to scale, and scales of different figures may be different.

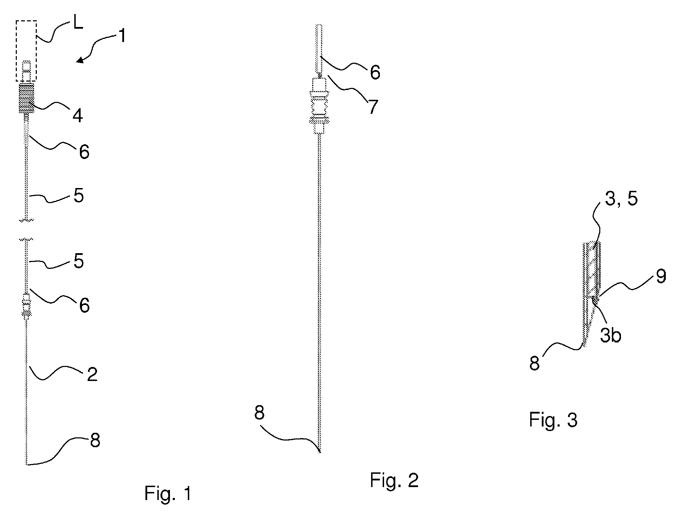

Fig. 1 shows an embodiment of a device 1 for irradiating human or animal tissue. The tissue may be nerves, in particular nerve tissue and nerve cells, particularly nociceptive nerve tissue or nociceptive cells.

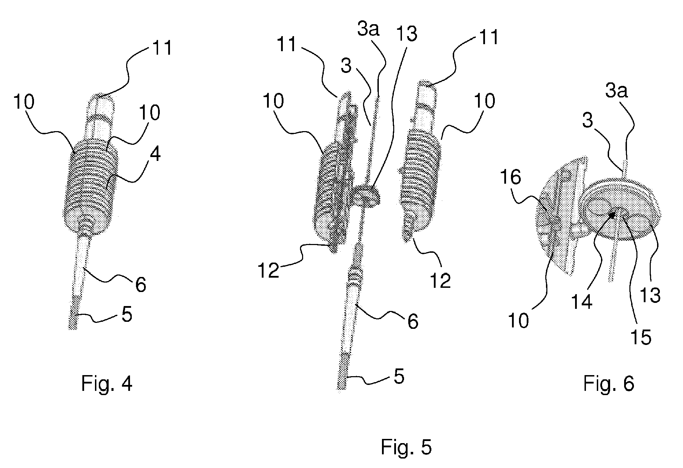

The device 1 comprises an applicator which in the present case is a spinal needle 2 of 25G type. Further, the device comprises a light guide presently represented by an optical fiber 3 (see Fig. 5 and Fig. 6). A first end 3a (see Fig. 5 and Fig. 6) of the optical fiber 3 is fixed in a shell 4, which is described in more detail in connection with Fig. 4 to 6.

The optical fiber 3 is surrounded by a sheathing 5 which connects between the shell 4 and an upper end of the needle 2. The sheathing 5 is designed such that laser light coupled into the optical fiber 3 can be optimally guided through.

In the present embodiment, shrink hoses 6 are applied to both ends of the sheathing 5 in order to ease fastening, in particular liquid tight fastening, thereof to the shell 4 and needle 2, respectively.

Fig. 2 shows the needle 2 in more detail, in particular the respective shrink hose 6. The shrink hose 6 and sheathing 5 are fixed to an upper end of a grip 7 of the needle 2 by gluing or other suitable means.

The optical fiber 3 is further guided through the needle 2, where a second end 3b of the optical fiber 3, as the case may together with the sheathing 5, is fixed near a tip cut 8 of the needle 2. The optical fiber 3 is positioned relative to the axial direction of the needle 2 such that an edge 9 of the tip of the needle 2 is flush with the second end 3b of the optical fiber 3, which can be seen in more detail in Fig. 3.

Fig. 4 shows the shell 4 with the sheathing 5 being connected thereto via the other one of the shrink hoses 6. The shell 4 comprises two half shells 10 in which the sheathing 5 and optical fiber 3 are fixed, which will be described in more detail below.

The shell 4 comprises a connector section 11 to which a laser light source L may be connected to, via a Luer-locking mechanism for example. The optical fiber 3 is fixed within the shell 4 such that laser light emitted by the laser light source L attached to the connector section 11 can be optimally coupled into the optical fiber 3. Note that the laser light source is schematically shown only in Fig. 1.

Fig. 5 in particular shows the shell 4 in an exploded view. As can be seen, half shell extensions 12 formed at lower ends of the half shells 10 clasp the respective shrink hose 6 as soon as the half shells 10 are joined together, via a snap mechanism, preferably of detachable type, for example. Hence a tight seal between the sheathing 5 and the shell 4 can be accomplished.

The optical fiber 3 is fixed and centered within the shell 4, inter alia via a mounting disc 13, which is also show in Fig. 6 in more detail. The mounting disc 13 comprises a center bore 14 with a stud hole type depression 15. The diameter of the center bore 14 is adapted to the diameter of the optical fiber 3 which may be 0.2 mm to 0.3 mm. Such fibers readily fit into the 25G type needle 2. The optical fiber 3 is fixed to the mounting disc 13 by filling the depression 15 with glue or something similar.

Fixtures 16 are provided in one or both half shells 10 such that the mounting disc 13, and thereby the optical fiber 3, can be attached within the shell 4 in a centered position in a groove provide for this purpose. Additional fixtures (not explicitely shown) provided at the upper end of the shell 4, e. g. in the area of the connector section 11, can be used to position, in particular to center, the first end 3a of the optical fiber 3 such that it can be easily and optimally coupled to a laser light exit site of the laser light source L.

As an alternative to the connector shown in Fig. 4 to 6 also another connector could be used for instance a SMA connector.

The device described in connection with Fig. 1 to Fig. 6 can be operated as follows:

First, tissue locations are determined to which laser light shall be applied to. Then the needle 2 is inserted into the human or animal body or tissue such that the tip end of the needle is positioned at or as close to the locations as required. During insertion of the needle 2, the tip cut 8 of the needle 2 opens and cuts tissue lying in front or ahead of the tip end or tissue to be irradiated. In using adequate needles 2, as for example spinal needles, even tissue lying comparatively deep within the human or animal body can be reached.

If the optical fiber 3 and, as the case may be, the sheathing 5 have not yet been introduced or embedded within the needle 2, the optical fiber 3, if so together with the sheathing 5, is introduced into the needle 2, such that the second end 3b of the optical fiber 3 flushes with the edge 9 of the needle 2. The shrink hose 6 close to the second end 3b, or other suitable stopper mechanisms, may be used to prevent the optical fiber 3 from being further introduced into the needle 2 as soon as the second end flushes with the edge 9.

Now the optical fiber 3 is in position for applying or irradiating laser light to the tissue in concern. Laser light is applied to the tissue by means of the optical fiber 3 in that laser light exiting an exit site of the laser light source L is coupled into the optical fiber 3. The laser light propagates within the optical fiber 3 and finally hits the tissue in concern.

The laser light source L can be connected to the connector section 11 before or after inserting the needle into the human or animal body and, as the case may be, after introducing the optical fiber 3 into the needle 2.

The laser light source L can be easily fixed to the connector section 11 if a Luer-locking mechanism is provided, for example. The connector section 11 and respective counterpart at the laser light source L are adapted such that upon establishing connection the laser light exit site is optimally positioned with respect to the first end 3a of the optical fiber 3.

Now the laser light source L, can be powered to emit laser light in a pulsed mode intermittent fashion with the following characteristics:
a) laser light wavelength in the range of 690 nm to 710 nm, preferably 700 nm to 705 nm, preferably 703 nm (+/- 2nm);
b) laser light source L average output power in the range of 0.5 mW to 10 mW, in particular 1 mW to 6 mW, in particular 4 mW to 6mW (+/- 0.8 mW);
c) laser light pulsewidth in the order of nano seconds or picoseconds, in particular about 25 ns;
d) laser light source output peak power in the range of 30 nW to 50 nW, in particular 40 nW (+/- 8nW);
e) laser light intermittent period between 1 s (seconds) and 30 s, in particular 5 s and 10 s.

The laser light propagates through the optical fiber 3 and hits the tissue in the region of the tip cut 8 positioned in the human or animal body. Hence, application of the laser light in this case represents a deep tissue low intensity laser therapy or treatment (DT-LILT). The laser light characteristics as set out above cause selective destruction of the tissue in concern via laser light absorption and cell resonance effects.

In particular, for example by using a wavelength of 703 nm, selective destruction of nociceptive nerves, in particular C-fibres, can be obtained while surrounding tissue is not or at least not substantially destroyed or affected. The latter is mainly due to the fact that the low intensity laser light does not generate sufficient heat to bring about heat induced tissue disruption.

Selective destruction of nerve or nociceptive tissue is probably based on the fact that this kind of tissue consists of lipids as predominant component which readily absorb laser light of the above identified wavelength, whereas surrounding tissue does not. Note that this is different from using heat for disrupting tissue, as is the case with known deep tissue laser treatment devices.

In destroying nociceptive nerves or nerve cells in this manner, a successful and long term pain release can be obtained. In analogy, similar results can be obtained if a deep tissue low intensity laser ablation (DT-LILA) or deep tissue low intensity laser neuroablation (DT-LILNA) is carried out.

Compared to existing deep tissue medical lasers today which disrupt tissue by generating heat and therefore bring about a non selective action destroying all the tissues on contact without any specificity, the proposed device 1 allows selective and gentle destruction in a long term time range of at least several months.

Also, compared to existing low level laser therapy (LLLT) devices, cell destruction is more efficient with the proposed device 1. This is due to the fact that known LLLT devices are non specific in their action, and destruction of nociceptive tissue, bringing about pain relief is obtained by indirect mechanisms most likely through heat generation, vaso-dilatation or by metabolic changes. The known LLLT devices do not cause specific death of nociceptive tissue, in particular pain nerves and only provide temporary pain relief.

A further advantage of the proposed device 1 is that DT-LILT can even be applied to specific areas of the human or animal spine, for example for long term pain relief.

Preliminary results on patients show that DT-LILT indeed selectively destroys pain nerves while other tissue in the area of treatment is left intact. In particular although no local anesthetic was used or any medications whether injectable or oral were used on the patients, all patients noted immediate and complete resolution of pain symptoms after treatment. Pain relief was about 100%, corresponding to a VAS pain measurement value of about zero or simply "No Pain". Further, sensing, tactile, pressure and motor functions were not affected and hence kept intact, indicating the selectivity of DT-LILT. Except for the long term effects, DT-LILT applied to pain nerves is comparable to injecting a large volume of local anesthetic into the area of treatment resulting in pain relief.

DT-LILT can for example be conducted with the help of X-ray views, in particular AP X-ray views. In particular it is possible to use computer tomographic views, in particular real-time views, during inserting the needle 2 and, if required, during applying the laser light. X-ray views can be used to navigate the needle 2, i. e. the tip cut 8, to the predefined tissue locations.

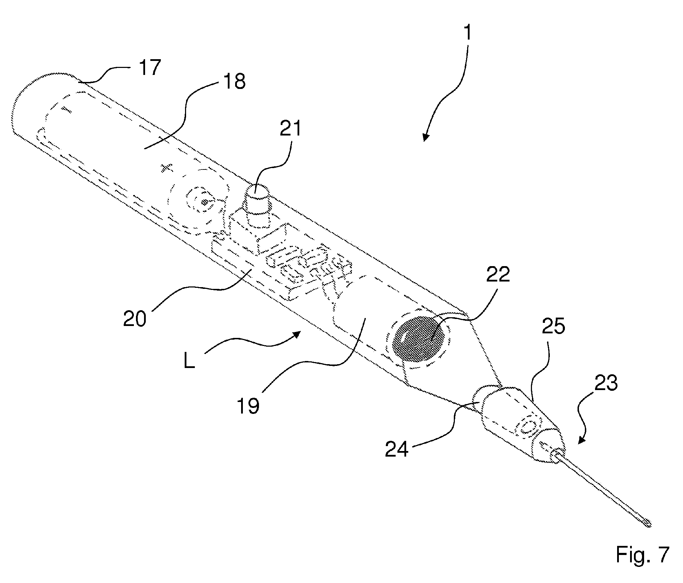

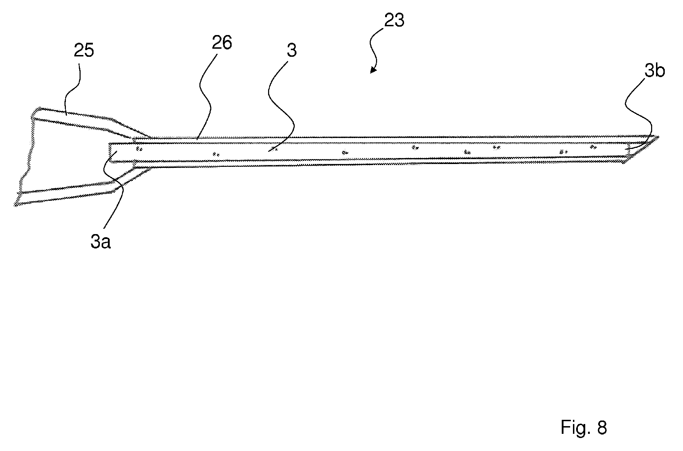

Due to the low intensity laser light needed, it is possible to power the laser light source L with a battery, particularly of rechargeable type. Especially in this case, at least the battery and laser light source L can be accommodated in a casing (as shown in more detail in Fig. 7 and 8) not requiring separate grounding during charging the battery or operating the device 1 or both.

Note that the terms DT-LILT, DT-LILNA and DT-LILA as well as DT-LIL (deep tissue low intensity laser) used above are pending trademark registrations before the US Patent and Trademark Office for entry to the Principal Register.

Fig. 7 shows in a transparent type representation another embodiment of a device 1 for irradiating tissue. The device 1 comprises a casing 17 accommodating therein an internal power supply, the source of energy which is a battery 18 in the present case, and the laser light source L, comprising one laser diode 19 in the present case. Note that the device 1 may comprise more than one laser light source L, in particular more than one laser diodes 19.

The battery 18 is connected to an electronic board 20 which is in particular adapted to power the laser diode 19 with energy provided by the battery 18. The electronic board 20 comprises a user interface, which in the present case comprises a button 21. The user interface may be more elaborate as described further above. Upon a user pressing the button 21, the laser diode 19 is activated to emit laser light. Upon releasing the button 21, the laser diode 19 stops emitting laser light. Any other switching modes may also be contemplated.

Optical elements 22 downstream the laser diode 19 are designed such that laser light emitted by the laser diode 19 is optimally coupled into or optimally focused towards an optical fiber 3 of an applicator 23 attached to the casing 17. The applicator 23 is attached to a connector section 24 provided in a face end side of the casing 17. The connector section 24 in the present case is adapted to hold a corresponding connector section 25 of the applicator 23 via friction locking. However, other locking mechanisms in the field may be used as well, as for example Luer-Locks and so on.

Fig. 8 shows in a cross-sectional view of the applicator 23. The applicator 23 comprises a cannula 26 fixed to the corresponding connector section 25. A light guide, in the present case the an optical fiber 3, is accommodated within the cannula 26, wherein the second end 3b of the optical fiber 3 is positioned near the tip end of the cannula 26. The first end 3a of the optical fiber 3 extends beyond the cannula 26 such that it enters a hole in the face end side of the connector section 24 in the course of connecting the applicator 23 to the casing 17. The first end 3a of the optical fiber 3 is positioned and adequately arranged in the hole such that laser light which is emitted by the laser diode 19 and passes the optical elements 22 is optimally coupled into the optical fiber 3. Light coupled into the optical fiber 3 can be used to treat or therapy tissue, as already described further above.

### Reference Signs

- 1: device
- 2: spinal needle
- 3: optical fiber
- 3a: first end
- 3b: second end
- 4: shell
- 5: sheathing
- 6: shrink hose
- 7: grip
- 8: tip cut
- 9: edge
- 10: half shell
- 11: connector section
- 12: half shell extension
- 13: mounting disc
- 14: center bore
- 15: depression
- 16: fixture
- 17: casing
- 18: battery
- 19: laser diode
- 20: electronic board
- 21: button
- 22: optical element
- 23: applicator
- 24: connector section
- 25: corresponding connector section
- 26: cannula

- L: laser light source

## Claims

1. Device (1) for irradiating human or animal tissue, in particular nerves, nerve tissue, and nerve cells, in particular nociceptive nerve cells, e.g. C-fibres, in human or animal tissue, with laser light, comprising
at least one laser light source (L, 19) adapted to generate laser light having a wavelength between 690 nm and 710 nm, in particular between 701 nm and 705 nm, preferably 703 nm;
a light guide (3) for guiding through laser light generated by at least one of the at least one laser light source (L, 19); wherein
a first end (3a) of the light guide (3) is coupled or adapted to being coupled or connected to a laser light exit site of at least one laser light source (L, 19); and
an applicator (2, 23), preferably of handheld-type, adapted to apply the laser light to the tissue,
the applicator (2, 23) containing, holding, being coupled, connected or connectable to at least a second end (3b) of the light guide (3).

2. Device (1) according to claim 1 wherein the light guide (3) comprises an optical fiber (3), preferably having a diameter between 0.15 mm and 0.7 mm, in particular between 0.15 mm and 0.5 mm, in particular in a range from 0.2 mm to 0.3 mm.

3. Device (1) according to claim 1 or 2, wherein the applicator (2, 23) comprises at least one hollow needle (2), preferably having a tip cut (8) to open or cut tissue lying in front of or ahead of the tissue to be irradiated, the needle (2) preferably having an inner diameter of less than 0.6 mm, preferably of less than 0.5 mm, the light guide (3) preferably having a diameter of 0.3 mm at the most and being inserted or insertable into the needle cannula, preferably being embedded therein, in particular fixed therein in a non-detachable manner, in particular by gluing.

4. Device (1) according to at least one of claims 1 to 3, at least one of the applicator (2, 23) and at least one laser light source (L, 19) comprising a locking or latching mechanism (11), in particular a Luer-locking mechanism, for fixing the light guide (3) and/or for attaching or detaching the applicator (2, 23) to the at least one laser light source (L, 19) and/or light guide (3).

5. Device (1) according to at least one of claims 1 to 4, the at least one laser light source (L, 19) being adapted to emit laser light with an output average power between 0.5 mW to 10 mW, in particular between 1 mW and 6 mW, preferably between 4 mW and 6 mW, preferably of 5 mW at the most, preferably a pulsed laser light with a pulse length in the order of nanoseconds to picoseconds or less, in particular 0.1 to 50 ns, preferably 25 ns, and/or an output peak power of preferably 30 nW to 50 nW.

6. Device (1) according to at least one of claims 1 to 5, further comprising a timer unit for respectively activating and inactivating at least one of the at least one laser light source (L, 19), in timing intervals in a range between 1 s to 30 s, in particular 3 s to 7 s.

7. Device (1) according to at least one of claims 1 to 6, further comprising an exchangeable and/or rechargeable source of energy (18), in particular a rechargeable battery source of energy (18), for powering at least one of the at least one laser light source (L, 19).

8. Device (1) according to at least one of claims 1 to 7, comprising a casing (17), preferably a non-conducting insulated casing (17), more preferably a casing (17) of handheld-type, accommodating therein at least one of the at least one laser light source (L, 19), source of energy (18) and optical coupling means (22) for effectively coupling laser light into the light guide (3).

9. Device (1) according to at least one of claims 1 to 8, further comprising a user interface (21), preferably comprising at least one button (21) and/or at least one touch-sensitive element and/or at least one display element, adapted to select and/or display at least one operational parameter of the device (1).

10. Device (1) according to at least one of claims 1 to 9, the applicator (2, 23) or second end (3b) of the light guide (3) being adapted to apply the laser light to an area of about the cross-section of the light guide (3), preferably with a scatter radius of 1 mm at the most.
